# EUROPEAN PATENT SPECIFICATION

(11) **EP 1 095 062 B1**
(45) Date of publication and mention of the grant of the patent: **13.09.2006**
(21) Application number: 99931969.2
(22) Date of filing: 28.06.1999
(51) Int. Cl.: C07K 16/28, G01N 33/48, C12Q 1/68

(54) **METHOD OF TREATING DISORDERS BY MODULATING THE INTERACTION OF NATURAL KILLER RECEPTORS ON T CELLS WITH THEIR RESPECTIVE LIGANDS**
METHODE ZUR BEHANDLUNG VON KRANKHEITEN DURCH VERÄNDERUNG DER WECHSELWIRKUNG ZWISCHEN NATURAL KILLER REZEPTOREN AUF T-ZELLEN MIT IHREN JEWEILIGEN LIGANDEN
PROCEDE DE TRAITEMENT DE TROUBLES PAR MODULATION DE L'INTERACTION DES RECEPTEURS TUEURS NATURELS SUR DES LYMPHOCYTES T ET LEURS LIGANDS RESPECTIFS

(30) Priority: 09.07.1998 US 92151 P; 25.11.1998 US 109894 P
(43) Date of publication of application: 02.05.2001
(73) Proprietor: Nickoloff, Brian J., Burr Ridge, IL 60527-8396 (US)
(72) Inventor: Nickoloff, Brian J., Burr Ridge, IL 60527-8396 (US)
(74) Representative: Hallybone, Huw George
(86) International application number: PCT/US1999/014549
(87) International publication number: WO 2000/002923

(56) References cited:
- US-A- 5 028 424
- US-A- 5 185 430
- US-A- 5 770 387
- CICCONE ERMANNO ET AL: "Self class I molecules protect normal cells from lysis mediated by autologous natural killer cells." EUROPEAN JOURNAL OF IMMUNOLOGY, vol. 24, no. 4, 1994, pages 1003-1006, XP009011692 ISSN: 0014-2980
- KAUFMAN D S ET AL: "Inhibition of selective signaling events in natural killer cells recognizing major histocompatibility complex class I." PROCEEDINGS OF THE NATIONAL ACADEMY OF SCIENCES OF THE UNITED STATES OF AMERICA. UNITED STATES 3 JUL 1995, vol. 92, no. 14, 3 July 1995 (1995-07-03), pages 6484-6488, XP002243412 ISSN: 0027-8424
- VITALE M ET AL: "Coexpression of two functionally independent p58 inhibitory receptors in human natural killer cell clones results in the inability to kill all normal allogeneic target cells." PROCEEDINGS OF THE NATIONAL ACADEMY OF SCIENCES OF THE UNITED STATES OF AMERICA. UNITED STATES 11 APR 1995, vol. 92, no. 8, 11 April 1995 (1995-04-11), pages 3536-3540, XP002243413 ISSN: 0027-8424
- NICKOLOFF B J ET AL: "Role of T-cells bearing receptors (CD94, CD158, CD161) for MHC class I and nonclassical MHC-like (CD1d) antigens in psoriasis." JOURNAL OF INVESTIGATIVE DERMATOLOGY, vol. 112, no. 4, April 1999 (1999-04), page 584 XP009011729 60th Annual Meeting of the Society for Investigative Dermatology;Chicago, Illinois, USA; May 5-9, 1999 ISSN: 0022-202X
- NICKOLOFF BRIAN J ET AL: "Response of murine and normal human skin to injection of allogeneic blood-derived psoriatic immunocytes: Detection of T cells expressing receptors typically present on natural killer cells, including CD94, CD158, and CD161." ARCHIVES OF DERMATOLOGY, vol. 135, no. 5, May 1999 (1999-05), pages 546-552, XP009011742 ISSN: 0003-987X
- MORETTA A ET AL: "P58 MOLECULES AS PUTATIVE RECEPTORS FOR MAJOR HISTOCOMPATIBILITY COMPLEX (MHC) CLASS I MOLECULES IN HUMAN NATURAL KILLER (NK) CELLS. ANTI-P58 ANTIBODIES RECONSTITUTE LYSIS OF MHC CLASS I-PROTECTED CELLS IN NK CLONES DISPLAYING DIFFERENT SPECIFICITIES" JOURNAL OF EXPERIMENTAL MEDICINE, TOKYO, JP, vol. 178, no. 2, August 1993 (1993-08), pages 597-604, XP009005922 ISSN: 0022-1007
- EXLEY M ET AL: "REQUIREMENTS FOR CD1D RECOGNITION BY HUMAN INVARIANT VALPHA24+ CD4-CD8-T CELLS" JOURNAL OF EXPERIMENTAL MEDICINE, TOKYO, JP, vol. 186, no. 1, 7 July 1997 (1997-07-07), pages 109-120, XP001152868 ISSN: 0022-1007
- EXLEY M ET AL: "CD161 (NKR-P1A) COSTIMULATION OF CD1D-DEPENDENT ACTIVATION OF HUMAN T CELLS EXPRESSING INVARIANT VALPHA24JALPHAQ T CELL RECEPTOR ALPHA CHAINS" JOURNAL OF EXPERIMENTAL MEDICINE, TOKYO, JP, vol. 188, no. 5, 7 September 1998 (1998-09-07), pages 867-876, XP001074739 ISSN: 0022-1007
- SUMIDA ET AL: "Selective Reduction of T Cells Bearing Invariant Valpha24JalphaQ Antigen Receptor in Patients with Systemic Sclerosis" JOURNAL OF EXPERIMENTAL MEDICINE, TOKYO, JP, vol. 182, no. 4, 1 October 1995 (1995-10-01), pages 1163-1168, XP002104249 ISSN: 0022-1007
- MIEZA M A ET AL: "SELECTIVE REDUCTION OF VALPHA14+ NK T CELLS ASSOCIATED WITH DISEASE DEVELOPMENT IN AUTOIMMUNE-PRONE MICE" JOURNAL OF IMMUNOLOGY, THE WILLIAMS AND WILKINS CO. BALTIMORE, US, vol. 156, 1996, pages 4035-4040, XP001159198 ISSN: 0022-1767
- NIEDA M ET AL: "ACTIVATION OF HUMAN VALPHA24NKT CELLS BY ALPHA-GLYCOSYLCERAMIDE IN A CD1D-RESTRICTED AND VALPHA24TCR-MEDIATED MANNER" HUMAN IMMUNOLOGY, NEW YORK, NY, US, vol. 60, no. 1, January 1999 (1999-01), pages 10-19, XP001146056 ISSN: 0198-8859
- BLUMBERG R S ET AL: "EXPRESSION OF A NONPOLYMORPHIC MHC CLASS I-LIKE MOLECULE CD1D BY HUMAN INTESTINAL EPITHELIAL CELLS" JOURNAL OF IMMUNOLOGY, vol. 147, no. 8, 1991, pages 2518-2524, XP001156220 ISSN: 0022-1767
- CARBONE ET AL: 'Natural Killer Clones Recognize Specific Soluble HLA Class I Molecules' EUROPEAN JOURNAL OF IMMUNOLOGY vol. 26, no. 3, 1996, pages 683 - 689, XP002923656
- LOPEZ-BOTET ET AL: 'The CD94/NKG2 C-Type Lectin Receptor Complex' IMMUNOLOGICAL RESEARCH vol. 16, no. 2, 1997, pages 175 - 185, XP002923657
- PHILLIPS ET AL: 'CD94 and A Novel Associated Protein (94AP) Form A NK Cell Receptor Involved in the Recognition of HLA-A, HLA-B and HLA-C Allotypes' IMMUNITY vol. 5, August 1996, pages 163 - 172, XP002923658
- LAZETIC ET AL: 'Human Natural Killer Cell Receptors Involved in MHC Class I Recognition are Disulfide-Linked Heterodimers of CD94 and NKG2 Subunits' JOURNAL OF IMMUNOLOGY vol. 157, 1996, pages 4741 - 4745, XP002923659
- MAJEWSKI ET AL: 'Defective Natural Killer- and Killer-Cell Activity Associated with Increased Polymorphonuclear Leukocyte Adherence in Psoriasis' ARCH. DERMATIOL. RESEARCH vol. 278, 1986, pages 264 - 269, XP002923660
- BROSSAY L. ET AL: 'CD1d-mediated recognition of an alpha-Galactosylceramide by natural killer T cells is highly conserved through mammalian evolution' J. EXP. MED. vol. 188, October 1998, pages 1521 - 1528, XP002996702

## Description

### RELATED APPLICATIONS

This application claims priority to U.S. provisional applications 60/092,151, filed July 9, 1998, and 60/109,894, filed November 25, 1998.

### BACKGROUND OF THE INVENTION

The present invention relates generally to a novel method of treating cutaneous as well as extracutaneous diseases, particularly psoriasis, and, more particularly, to a method of inhibiting various types of diseases by targeting immunological reactions whereby T lymphocytes expressing surface molecules typically expressed by natural killer cells (i.e., NK-T cells) become activated through interaction of these surface receptors with their respective ligands. An Example of such a receptors is CD161. This receptor recognizes MHC-class I-like antigens such as CD1d, but may also include other activating molecules.

Psoriasis is a common, chronic, inherited skin disorder triggered when activated immunocytes infiltrate the epidermis and establish a Th-1 type cytokine network culminating in keratinocyte hyperplasia and angiogenesis (reviewed in 1). The precise mechanism responsible for the initial or sustained immunocyte activation in skin is not known. Investigators are also attempting to discern the genetic basis for psoriasis, and ultimately the gene or genes responsible for causing the disease will be linked either directly or indirectly to immunocyte activation since it is clear from a number of clinical and basic research laboratories that psoriasis is dependent on the immune system for its initial appearance and prolonged appearance in the skin (reviewed in 2).

Recently, a rare type of memory T cell that also expressed receptors on natural killer cells (i.e., so-called NK-T cells) was discovered in the epidermis of acute and chronic psoriatic plaques (3,4). While these immunocytes had been previously overlooked in the human skin, other investigators had identified them in the blood, bone marrow, spleen and thymus in various rodents. The NK-T cells in mice were predominantly characterized as having an invariant T cell receptor, and gained further recognition in an immunoregulatory context involving both autoimmune as well as inflammatory and neoplastic disorders (5-8). Very little was previously known regarding the presence, function, or significance of NK-T cells in human subjects. Whether NK-T cells were functioning in a direct (pathogenic) or indirect (immunoregulatory) context in human disease was also not previously explored in human subjects.

Many treatments are available to treat psoriasis that focus on the immune system. However, none of the widely used immunosuppressive therapies interrupt a specific ligand-receptor pathway. For example, treatments such as cyclosporin A, corticosteriods or ultraviolet light act in a non-specific fashion to turn off lymphocyte activation with a "shot-gun" like effect. Since such general immunosuppressive strategies are accompanied by side effects, including risk for adrenal suppression, opportunistic infection or neoplasms, more highly targeted treatments are clearly indicated. By focusing specifically on the NK-T cell surface molecules, such as CD161, or their respective ligands, this invention provides a much needed and novel treatment approach to psoriasis, as well as other NK-T cell mediated disorders.
D1 (Nickoloff, B.J. et al. (1999) Journal of Investigative Dermatology, vol. 112, 584) discusses the immunologic and genetic basis for psoriasis. T-cells bearing NKRs for both classical MHC antigens and CD1d were shown to be present in lesions.
D3 (Exley, M. et al. (1998) Journal of Experimental Medicine, vol. 188, 546-552) describes the use of anti-CD1d and anti CD161 antibodies for immunostaining and D4 (Exley, M. et al. (1997) Journal of Experimental Medicine, vol. 186, 109-120) describes the use of the anti-CD1d antibody for blocking CD161 positive T-cell proliferation.
D5 (Brossay L. et al., (1998) Journal of Experimental Medicine, vol. 188, 1521-1528) also describes the use of an anti-CD1d antibody for blocking NKT-cell proliferation.

### BRIEF SUMMARY OF THE INVENTION

As described herein, the inventor has discovered the presence of NK-T cells in psoriasis, characterized the expression of their surface receptors and respective ligands, and demonstrated that NK-T cells are pathogenic for psoriasis. A novel pathophysiological basis for psoriasis was established in which NK-T cells bearing the surface receptor CD161 interact with MHC class I-like (i.e., CD1d) on the surface of antigen presenting cells such as keratinocytes (2). Described herein are methods of inhibiting the interaction of NK-T cell receptors with their respective ligands, thereby interfering with the stimulation of the NK-T cells. Such inhibition of this molecular interaction and of the subsequent resulting stimulation, prevents, reverses or reduces the formation of psoriasis.

As used herein, the term "novel ligand/receptors" refers to the NK-T cell receptor CD161, (and any and all homologs) as well as its respective ligands including non-classical MHC class I-like molecules such as CD1d.

Furthermore, described herein are methods of treating cutaneous and extracutaneous disorders by inhibiting the interaction of the NK-T cell receptor CD161, with its respective ligands. Besides psoriasis, diseases that can be treated with this invention include several benign and malignant immunologically-mediated disorders such as: atopic dermatitis, alergic and irritant contact dermatitis, lupus erythematosus, lichen planus, drug-induced dermatitides, alopecia areata, androgenetic alopecia, and autoimmune blistering disorders.

### BRIEF DESCRIPTION OF THE DRAWING

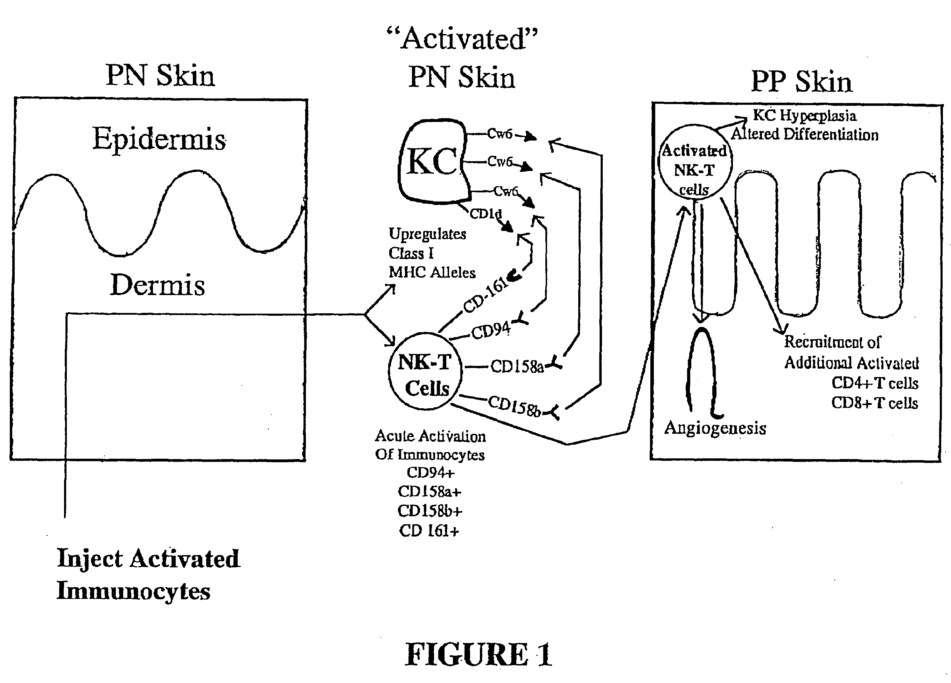

**FIG. 1.** Illustration of a proposed mechanism by which activated immunocytes induce psoriasis.

### DETAILED DESCRIPTION OF THE INVENTION

Psoriasis, a common and inherited skin disease, can be transferred through immunocytes using a novel animal model experimental system developed by the inventor (9). Thus symptomless (PN) skin engrafted onto SCID mice may be induced to become a full fledged psoriatic plaque by injection of activated immunocytes (10). To further understand the local immune reactions taking place in the injected skin grafts, the inventor examined for acute activation markers such as CD69 and CD25. As there was acute reactivation taking place, the inventor began a search for the molecular basis for such immunological activation (11).

Moreover, not only was psoriasis being created using autologous combinations of skin and immunocytes, but also in allogenic settings as well (3). This unexpected finding led the investigator to consider the involvement of nonconventional immunocytes such as NK-T cells.

To explore the possible involvement of NK-T cells, the inventor began immunostaining of cryostat sections obtained from the grafts before and immediately after injection of activated immunocytes and discovered the presence of T cells bearing these receptors (i.e., CD161). Although the ligand for CD161 (CD1d) was previously believed to be restricted to the thymus and intestinal tract (2), the inventor discovered that psoriatic lesions had overexpression of CD1d as well (3).

Thus, these SCID mouse model based discoveries facilitated the recognition of these previously overlooked immunocytes and one of their important ligands (i.e., CD1d). The interaction between immunocytes bearing CD161 with the epidermal cells bearing CD1d produced an immunological reaction that did not include any killing or cytopathic effect (graft versus host disease) on the keratinocytes. Rather the interaction triggered keratinocyte and endothelial cell hyperplasia. While not limiting this invention, the most probable mechanism of induction of psoriasis is illustrated in FIG. 1.

The Examples herein demonstrate the role of NK-T cells in psoriasis and, particularly, the role of activation of such cells via interaction of receptors on the surface of the cells (such as CD161) with their respective ligands in the disease. The Examples are briefly summarized below:

### Example 1:

Having established the functional role of the aforementioned NK-T cells, the inventor next demonstrated that other NK-T cell receptor/ligands such as CD161 and CD1d were involved in psoriasis. An extensive screening of normal and various other cutaneous disease processes, including psoriasis, revealed that the functional homologs of CD94/MHC class I antigens such as CD161 and CD1d were also present in acute and chronic lesions of psoriasis. As mentioned earlier, the NK-T cells could participate in both direct (pathogenic), as well as indirect (immunoregulatory) roles. To further demonstrate the role NK-T cells in psoriasis, the inventor determined if a CD161 NK-T cell line could cause psoriasis using the SCID mouse model.

### Example 2:

The blood of a psoriatic patient was exposed for several weeks to repeated stimulation with bacterial superantigens and IL-2. A NK-T cell line was produced and characterized, and revealed it to be a CD3+ and CD4+ cell line that also expressed CD161. Upon injection into PN skin, this line produced a full-fledged psoriatic plaque in which CD161 positive NK-T cells were juxtaposed to CD1d expressing keratinocytes

To verify that this CD161+ NK-T cell line could recognize keratinocyte CD1d, cultured multi-passage keratinocytes were pre-incubated with IFN-gamma (to up-regulate their CD1d), and then co-incubated with the NK-T cell line. While normal keratinocytes with low or absent CD1d did not produce NK-T cell proliferation or cytokine release, when the pre-treated keratinocytes were mixed with NK-T cells, the NK-T cells became activated by forming clusters, incorporating tritiated thymidine and secreting large amounts of the Th-1 cytokine (IFN-gamma), but not the Th-2 type cytokine IL-4.

This activation was dependent on CD1d recognition as addition of a blocking antibody against CD1d blocked the proliferation and cytokine release. The production of IFN-gamma is of particular note, because it has already been established that injection of IFN-gamma into the symptomless skin of patients can trigger the cellular immune response necessary to create psoriasis (2).

Thus, the Examples demonstrate not only that blocking the interaction of natural killer receptors on NK-T cells with their respective ligands prevents psoriasis but also that NK-T cells are directly pathogenic and that CD161 hearing NK-T cells can recognize and become activated by CD1d positive keratinocytes.

### NK-T Cell Receptors

Previously, receptors for class I-like molecules, such as CD161, were predominantly the focus of investigators interested in natural killer cell activity. In general, there are two broad classes of such receptors (12) including those receptors which contain a lectin-like domain (such as CD94 or CD161), and those that contain immunoglobulin (IG) superfamily-like domains (such as CD158a or CD158b).

The inventor has determined that immunocytes, and more particularly NK-T cells, are involved in the pathophysiology of psoriasis, and that blocking the pathway involving these novel ligand/receptor interactions will improve and prevent psoriatic plaques. By improve, it is meant that psoriasis is reduced at least about 20% in a treated patient as compared to an untreated patient. In preferred embodiments, psoriasis is reduced by at least about 50% in a treated patient as compared to an untreated patient Of course, reductions in psoriasis in treated patients of about 25%, 30%, 35%, 40 %, 45%, 55%, 60%, 65%, 70%, 75%, 80%, 85%, 90%, 95%, and 100% over untreated patients are considered improvements also.

To determine reduction, one may look at a number of psoriatic symptoms that include but are not limited to, decreased thickness of psoriatic plaque, decreased circumference of psoriatic plaque, amount of scaliness of the plaque, redness, and decreased pruritus.

Since CD161 is a lectin type C receptors, carbohydrate modifications of certain class I alleles, glycolipids that may bind to the class I or non-classical molecules such as CD1d, or signal sequence derived peptides may also be targeted by the methods disclosed herein.

It has been demonstrated that certain alleles within class I MHC molecules such as the amino acid of positions 77 and 80 of the alpha-1 helix influence recognition by relevant NK-receptors (13). Furthermore, no bound peptides were necessary, and another group has epidemiological data implicating a change in amino acid residue 73 of HLA-C which is strongly associated with psoriasis (14). It has also been recognized that a transmembrane component of HLA-Cw6 (cys-309) may influence recognition by NK receptors, and CD1d also has a comparable amino acid in its transmembrane domain as well (15).

One report (16) has observed genetic linkage on chromosome #1 near the region where CD1d is encoded. However, these investigators did not recognize the link to CD1d. The inventor contemplates that the genetic linkage may be due to a mutation in the CD1d which resides on chromosome #1. Thus, both residues exposed on the surface, or in the transmembrane domains of the MHC class I, or non-classical MHC-like antigen, can influence recognition by relevant NK-T cells, Therefore, within the scope of this invention is the use of compounds that interfere with the ability of NK-T cell receptors to recognition specific residues of MHC class I or class I-like conformations.

In a preferred embodiment, antibodies specific for a receptor that recognizes a class I-like molecule (such as CD1d) known to be associated with psoriasis are used. Methods of treating patients with antibodies or antibody fragments are known in the art (U.S. Patent No. 5,914,110). Peptides (naturally produced or chemically synthesized) that prevents or reduce the interaction of one or more NK-T cell receptor with its respective ligands may be used.

### MHC Class I Molecules

The present invention utilizes the ability to prevent or treat cutaneous or extracutaneous diseases by blocking the interaction of NK-T cell receptors and MHC class I molecules. Besides the 3 classical MHC class I molecules (HLA-A, -B, and -C), there are also nonclassical MHC molecules (CD1d) that may be involved in recognition by receptors on NK cells. Examples of inhibitory reactions mediated by such non-classical MHC molecules include HLA-G and HLA-E (17,18). The primary NK surface receptor molecule that interacts with HLA-E (and signal sequences derived from HLA-A, B, C) is the CD94 molecule. The primary NK surface receptor that interacts with CD1d is CD161.

In a preferred embodiment, antibodies specific for a class I-like molecule (*e.g.,* CD1d) associated with psoriasis are used to block the interaction of these molecules with NK-T cell receptors. The role of MHC class I molecule - NK-T cell interaction described herein explains the historic disease susceptibility loci for psoriasis; see Table I (19-23). The inventor contemplates that antibodies to essentially any of these molecules may be used in the present invention.

**Table I. Association Of HLA Antigens With Psoriasis**

| **References** | **Year** | **HLA antigens** |
|---|---|---|
| Russell et al. | 1972 | B13 [A13], B17 [w17] |
| White et al. | 1972 | B13 [A13], B17 [A17] |
| Seignalet et al. | 1974 | B13 [A13], B17 [A17] |
| Schunter & Schieferstein | 1974 | B13 [A13], B17 [A17] |
| Svejgaard et al. | 1974 | B13, B17 |
| Woodrow et al. | 1975 | B13 [A13], B17 [A17] |
| Krulig et al | 1975 | B13 [w16, B17 [w17] |
| Karvonen | 1975 | B13, Bw37 |
| Zachariae et al. | 1977 | B13, B17, B27 Bw37 |
| Gazit et al. | 1978 | B13, B17, B37, Cw6 |
| Brenner et al. | 1978 | B13, B17, B37 Cw6 |
| Hawkins et al | 1980 | B13, Bw57 (B17), Bw58(B17),Bw39(16), Cw6, DR7 |
| Tiilikainen et al | 1980 | B13, Bw57(B17), Cw6, Dr7 |
| Tiwari & Terasaki | 1981 | Dr7 |
| Henseler & Christophers | 1985 | Cw6 |
| Ozawa et al. | 1988 | B13, Bw37, Bw57(B17), Cw6, Cw2, Dr7, Cw11 |
| Nakagawa et al. | 1990 | Cw11 |
| Christophers & Henseler | 1990 | A30, B13, Bw57, Cw6 |
| Asahina et al. | 1991 | Cw9 |
| Nakagawa et al. | 1991 | A1, A2, B39, Bw46, Cw6, Cw7 Cw11 |
| Schmitt Egenolf et al. | 1993 | Cw7-B57-DRB1*0701/02(DR7)-DQA1*0201-ADQB1*0303 {DQw9(w3)} |
| Jenisch et al. | 1995 | DRB11*0701-DQA1*0201-DQB1*0303 |

Modified from Henseler T, Christophers E. HLA and psoriasis. In: Dubertret L, editor Psoriasis. Breschia: ISED; 1994. P. 10-13.*Antigens presented in square brackets are the HLA designations found in the reference, which have been replaced by the newer designations listed. Antigens listed in parenthesis are the "parent" antigens of the indicated "split" antigens. Antigens presented in braces are the HLA determinants of the listed HLA alleles. Real haplotypes are combined with a hyphen. A description of HLA antigen nomenclature is provided in Ref. #22. The references enumerated in the table are incorporated herein by reference.

The strongest linkage to certain MHC class I alleles can be understood by the relative ability of various class I alleles to serve as the appropriate ligand to differentially stimulate NK-T cells bearing their receptors, i.e. CD94, CD 158a, CD158b, CD161 (See Table II). In other words, if you express HLA-Cw6 (or other relevant MHC molecules), this surface molecule will trigger an immunological reaction mediated by immunocytes expressing CD94, CD158a, CD158b, or CD161.

**Table II: Relationship between HLA, class I receptors and Various HLA Antigens.**

| **HLA class I Receptors and HLA-class I-like Receptor** | **HLA Antigens Recognized by Natural Killer Receptors including non-classical CD1d** |
|---|---|
| CD94 | HLA-A, B, C signal sequence derived peptides combined with HLA-E |
| CD158a | Cw2, Cw5, Cw6 |
| CD158b | Cw1, Cw3, Cw7, Cw8 |
| CD161 | CD1d |

Of course, there are likely to be other examples of such ligand: receptor interactions that also function in a similar fashion as described above for class I MHC and CD94/CD158a/CD158b/CD161. There are other related non-classical type molecules and/or other family members of both the lectin-like type and immunoglobulin superfamily type.

### Methods for Preparing Antibody Compositions

In an embodiment of the present invention, antibodies are used to block the interaction of a novel ligand/receptor. Besides commercially available antibodies (some of which are enumerated herein), antibodies may be developed to a novel ligand/receptor and used in the present invention.

The antibody compositions for use in the present invention may be produced by a number of methods. Means for preparing and characterizing antibodies are well known in the art (See *e*.*g*., Harlow and Lane, 1988). Reference to antibodies throughout the specification includes whole polyclonal and monoclonal antibodies (mAbs), and parts thereof, either alone or conjugated with other moieties. Antibody parts include Fab and F(ab)₂ fragmented and single chain antibodies. The antibodies may be made *in vivo* in suitable laboratory animals, non-laboratory animals (U.S. Patent No. 5,891,698) or *in vitro* using recombinant DNA techniques (*e.g*., U.S. Patent Nos. 4,975,369 and 5,225,539). The antibodies may be humanized (U.S. Patent Nos. 5,891,996 and 5,889,157).

Briefly, a polyclonal antibody of the present invention is prepared by immunizing an animal with an immunogen comprising a novel ligand/receptor or peptide derived therefrom and collecting antisera from that immunized animal. A wide range of animal species can be used for the production of antisera. Typically an animal used for production of antisera is a sheep, a donkey, a chicken, a rabbit, a mouse, a rat, a hamster, a goat, or a guinea pig. Because of the relatively large blood volume of rabbits, a rabbit is a preferred choice for production of polyclonal antibodies.

As is well known in the art, a given composition may vary in its immunogenicity. It is often necessary therefore to boost the host immune system, as may be achieved by coupling a peptide or polypeptide immunogen to a carrier. Exemplary and preferred carriers are keyhole limpet hemocyanin (KLH) and bovine serum albumin (BSA). Other albumins such as ovalbumin, mouse serum albumin or rabbit serum albumin can also be used as carriers. Means for conjugating a polypeptide to a carrier protein are well known in the art and include glutaraldehyde, *m*-maleimidobenzoyl-N-hydroxysuccinimide ester, carbodiimide and bis-diazotized benzidine.

Antibodies, both polyclonal and monoclonal, specific for novel ligand/receptor may be prepared using immunization techniques, as are generally known to those of skill in the art. A composition containing antigenic epitopes of the polypeptide of interest is used to immunize one or more experimental animals, such as a rabbit or mouse, which will then proceed to produce specific antibodies against the polypeptide. Polyclonal antisera may be obtained, after allowing time for antibody generation, by bleeding the animal and preparing serum samples from the whole blood.

In preferred embodiments, the antibodies for use in the present invention are further purified by a process known as affinity purification. Methods of affinity purification of antisera, or other antibody compositions, are well known to those of skill in the art. Generally, an antibody composition is subjected to chromatography wherein the antigen is coupled to the resin of a column. Antibodies that are immunoreactive with the antigen are retained in the column, whereas antibodies (and other proteins) that are not immunoreactive with the antigen flow through. The immunoreactive antibodies are then eluted from the column. Elution may be by a number of methods. One such method includes the addition of 100% ethylene glycol to the column.

The amount of immunogen composition used in the production of polyclonal antibodies varies upon the nature of the immunogen, as well as the animal used for immunization. A variety of routes can be used to administer the immunogen (subcutaneous, intramuscular, intradermal, intravenous and intraperitoneal). The production of polyclonal antibodies may be monitored by sampling blood of the immunized animal at various points following immunization. A second, booster injection, also may be given. The process of boosting and titering is repeated until a suitable titer is achieved. When a desired level of immuneogenicity is obtained, the immunized animal can be bled and the serum isolated and stored, and/or the animal can be used to generate mAbs. One of the aspects of the present invention is a polyclonal sera that is relatively homogenous with respect to the specificity of the antibodies therein. Typically, polyclonal antisera is derived from a variety of different "clones." *i.e.,* B cells of different lineage; mAbs, by contrast, are defined as coming from antibody-producing cells with a common B-cell ancestor, hence their "mono" clonality.

mAbs may be readily prepared through use of techniques such as those exemplified in U.S. Patent 4,196,265. Typically, the production of mAbs involves immunizing a suitable animal with a selected immunogen composition, e.g., a purified or partially purified protein, polypeptide or peptide. The immunization composition is administered in a manner effective to stimulate antibody producing cells. Rodents such as mice and rats are preferred animals, however, the use of rabbit, sheep or frog cells is also possible. The use of rats may provide certain advantages, but mice are preferred, with the BALB/c mouse being most preferred as this is most routinely used and generally gives a higher percentage of stable fusions.

Following immunization, somatic cells with the potential for producing antibodies, specifically B lymphocytes (B cells), are selected for use in the mAb generating protocol. These cells may be obtained from biopsied spleens, tonsils or lymph nodes, or from a peripheral blood sample. Spleen cells and peripheral blood cells are preferred, the former because they are a rich source of antibody-producing cells that are in the dividing plasmablast stage, and the latter because peripheral blood is easily accessible. Often, a panel of animals will have been immunized and the spleen of animal with the highest antibody titer will be removed and the spleen lymphocytes obtained by homogenizing the spleen with a syringe. Typically, a spleen from an immunized mouse contains approximately about 5 x 10⁷ to about 2 x 10⁸ lymphocytes.

The antibody-producing B lymphocytes from the immunized animal are then fused with cells of an immortal myeloma cell, generally one of the same species as the animal that was immunized. Myeloma cell lines suited for use in hybridoma-producing fusion procedures preferably are non-antibody producing, have high fusion efficiency, and enzyme deficiencies that render them incapable of growing in certain selective media which support the growth of only the desired fused cells (hybridomas).

Any one of a number of myeloma cells may be used, as are known to those of skill in the art. For example, where the immunized animal is a mouse, one may use P3-X63/Ag8, X63-Ag8, 653, NS1/1, Ag4 1, Sp210-Ag14, FO, NSO/U, MPC-11, MPC11-X45-GTG 1.7 and S194/5XX0 Bul. For rats, one may use R210.RCY3, Y3-Ag 1.2.3, IR983F and 4B210. U-266, GM1500-GRG2, LICR-LON-HMy2 and UC729-6 are all useful in connection with human cell fusions.

One preferred murine myeloma cell is the NS-1 myeloma cell line (also termed P3-NS-1-Ag4-1), which is readily available from the NIGMS Human Genetic Mutant Cell Repository by requesting cell line repository number GM3573. Another mouse myeloma cell line that may be used is the 8-azaguanine-resistant mouse murine myeloma SP2/O non-producer cell line.

Methods for generating hybrids of antibody-producing spleen or lymph node cells and myeloma cells usually comprise mixing somatic cells with myeloma cells in a 2:1 ratio, though the ratio may vary from about 20:1 to about 1:1, respectively, in the presence of an agent or agents (chemical or electrical) that promote the fusion of cell membranes. Fusion methods using Sendai virus have been described, and those using polyethylene glycol (PEG), such as 37% (v/v) PEG. The use of electrically induced fusion methods is also appropriate.

Fusion procedures usually produce viable hybrids at low frequencies, about 1 x 10⁻⁶ to about 1 x 10⁻⁸. However, this does not pose a problem, as the viable, fused hybrids are differentiated from the parental, unfused cells (particularly the unfused myeloma cells that would normally continue to divide indefinitely) by culturing in a selective medium. The selective medium is generally one that contains an agent that blocks the *de novo* synthesis of nucleotides in the tissue culture media. Exemplary and preferred agents are aminopterin, methotrexate, and azaserine. Aminopterin and methotrexate block *de novo* synthesis of both purines and pyrimidines, whereas azaserine blocks only purine synthesis. Where aminopterin or methotrexate is used, the media is supplemented with hypoxanthine and thymidine as a source of nucleotides (HAT medium). Where azaserine is used, the media is supplemented with hypoxanthine.

The preferred selection medium is HAT. Only cells capable of operating nucleotide salvage pathways are able to survive in HAT medium. The myeloma cells are defective in key enzymes of the salvage pathway, *e*.*g*., hypoxanthine phosphonibosyl transferase (HPRT), and they cannot survive. The B-cells can operate this pathway, but they have a limited life span in culture and generally die within about two weeks. Therefore, the only cells that can survive in the selective media are those hybrids formed from myeloma and B-cells.

This culturing provides a population of hybridomas from which specific hybridomas are selected. Typically, selection of hybridomas is performed by culturing the cells by single-clone dilution in microtiter plates, followed by testing the individual clonal supernatants (after about two or three weeks) for the desired reactivity. The assay should be sensitive, simple and rapid, such as radioimmunoassays, enzyme immunoassays, cytotoxicity assays, plaque assays, dot immunobinding assays, and the like.

The selected hybridomas would then be serially diluted and cloned into individual antibody-producing cell lines, which clones can then be propagated indefinitely to provide mAbs. The cell lines may be exploited from mAb production in two basic ways. A sample of the hybridoma can be injected (often into the peritoneal cavity) into a histocompatible animal of the type that was used to provide the somatic and myeloma cells for the original fusion. The injected animal develops tumors secreting the specific mAb produced by the fused cell hybrid. The body fluids of the animal, such as serum or ascites fluid, can then be tapped to provide mAbs in high concentration. The individual cell lines could also be cultured *in vitro,* where the mAbs are naturally secreted into the culture medium from which they can be readily obtained in high concentrations. mAbs produced by either means may be further purified, if desired, using filtration, centrifugation and various chromatographic methods such as HPLC or affinity chromatography.

Whether polyclonal or monoclonal, antibodies to a novel ligand/receptor may be characterized for their ability to block the interaction of the novel ligand/receptors as described in the Examples herein.

### NK-T Cell Associated Diseases

Besides psoriasis, the methods of the present invention also include treatment of other cutaneous or extracutaneous disorders or diseases including: atopic dermatitis, allergic contact dermatitis, irritant contact dermatitis, lupus erythematosus, lichen planus, various drug-induced dermatitides, alopecia areata, androgenetic alopecia, and autoimmune blistering disorders (bullous pemphigoid or pemphigus vulgaris).

It should be noted that the successful management of the skin lesions of psoriatic plaques will improve the arthritis that can accompany such skin lesions. It is generally believed the arthritis (which is rheumatoid factor unrelated) associated with psoriasis results from a "spill-over" effect of cytokines and other inflammatory mediators from the skin into the adjacent synovial tissue. Thus, agents designed to interrupt the NK-T cells activation pathway in skin will also lead to improvement (i.e., disappearance) of the arthritic component that can produce significant morbidity.

Because of the similarity in clinical features and immunopathogenic pathways shared by psoriasis and several other autoimmune diseases (1) such as multiple sclerosis, diabetes mellitus, rheumatoid arthritis, and inflammatory bowel disorders (Crohn's disease and ulcerative colitis) this therapeutic approach will also be successful in the treatment of these common human diseases that are linked to certain HLA alleles, and in which activated NK-T cells mediate the disease.

It is also clinically well established that psoriatic patients have significantly reduced incidence of skin infections and tumors. Thus agents that can enhance the interaction of one or more NK-T cell receptors with their respective ligands are indicated for treatment of cutaneous infections and neoplasms in non-psoriatic patients. Alternatively, compounds that are capable of facilitating the binding or enhancing functional interactions involving NK-T cell receptors such that binding leads to activation of these immunocytes may be used to treat infectious and neoplastic assaults.

Since NK-T cells may bridge the innate and acquired immune system, it is possible that the reason psoriasis is so prevalent is because these patients possess these NK-T cells which can rapidly and effectively combat infections and neoplasms. The formation of plaques may be the price these individuals pay to be able to so effectively resist potentially deadly infections or metastatic skin cancers.

### Pharmaceutically Acceptable Carriers

In certain embodiments of the present invention compositions comprising compounds capable of altering the activation of NK-T cells are administered to a patient. The compound may be dissolved or dispersed in a pharmaceutically acceptable carrier or aqueous medium. Aqueous compositions of gene therapy vectors expressing novel receptor/ligands, mutants thereof, or antisense nucleic acids are also contemplated. The phrases "pharmaceutically or pharmacologically acceptable" refer to molecular entities and compositions that do not produce an adverse, allergic or other untoward reaction when administered to an animal, or a human, as appropriate.

As used herein, "pharmaceutically acceptable carrier" includes any and all solvents, dispersion media, coatings, antibacterial and antifungal agents isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

For human administration, preparations should meet sterility, pyrogenicity, general safety and purity standards as required by FDA Office of Biologics standards.

The biological material should be extensively dialyzed to remove undesired small molecular weight molecules and/or lyophilized for more ready formulation into a desired vehicle, where appropriate. The active compounds will then generally be formulated for parenteral administration, *e.g.*, formulated for injection via the intravenous, intramuscular, subcutaneous, intralesional, or even intraperitoneal routes.

The preparation of an aqueous composition that contains a compounds capable of altering the activation of NK-T cells as an active component or ingredient will be known to those of skill in the art in light of the present disclosure. Typically, such compositions can be prepared as injectables, either as liquid solutions or suspensions; solid forms suitable for using to prepare solutions or suspensions upon the addition of a liquid prior to injection can also be prepared; and the preparations can also be emulsified.

The pharmaceutical forms suitable for injectable use include sterile aqueous solutions or dispersions; formulations including sesame oil, peanut oil or aqueous propylene glycol; and sterile powders for the extempraneous preparation of sterile injectable solutions or dispersions. In all cases the form must be sterile and must be fluid to the extent that easy syringability exists. It must be stable under the conditions of manufacture and storage and must be preserved against the contaminating action of microorganisms, such as bacteria and fungi.

Solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

A protein, polypeptide, antibody, agonist or antagonist for use in the methods of the present invention can be formulated into a composition in a neutral or salt form. Pharmaceutically acceptable salts, include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, an liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

In terms of using polypeptide therapeutics as active ingredients, the technology of U.S. Patents 4,6-8,251; 4,601,903; 4,559,231; 4,559,230; 4,596,792; and 4,578,770 may be used.

The preparation of more, or highly, concentrated solutions for direct injection is also contemplated, where the use of DMSO as solvent is envisioned to result in extremely rapid penetration, delivering high concentrations of the active agents to a small tumor area.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intrperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion, (see for example, "Remington's Pharmaceutical Sciences" 15^{th} Edition, pages 1035-1038 and 1570-1580). Some variation in dosage will necessarily occur depending on the condition of the subject being treated. The person responsible for administration will, in any event, determine the appropriate dose for the individual subject.

The active polypeptides or agents may be formulated within a therapeutic mixture to comprise about 0.0001 to 1.0 milligrams, or about 0.001 to 0.1 milligrams, or about 0.1 to 1.0 or even about 10 milligrams per dose or so. Multiple doses can also be administered.

In addition to the compounds formulated for parenteral administration, such as intravenous or intramuscular injection, other pharmaceutically acceptable forms include, e.g., tablets or other solids for oral administration; liposomal formulations; time release capsules; and any other form currently used, including cremes.

One may also use nasal solutions or sprays, aerosols or inhalants in the present invention. Nasal solutions are usually aqueous solution designed to be administered to the nasal passages in drops or sprays. Nasal solutions are prepared so that they are similar in many respects to nasal secretions, so that normal ciliary action is maintained. Thus, the aqueous nasal solution usually are isotonic and slightly buffered to maintain a pH of 5.5 to 6.5.

In addition, antimicrobial preservatives, similar to those used in ophthalmic preparations, and appropriate drug stabilizers, if required, may be included in the formulation. Various commercial nasal preparations are known and include, for example, antibiotics and antihistamines and are used for asthma prophylaxis.

Additional formulatins which are suitable for other modes of administration include vaginal suppositories and pessaries. A rectal pessary or suppository may also be used.

Suppositories are solid dosage forms of various weights and shapes, usually medicated, for insertion into the rectum, vagina or the urethra. After insertion, suppositories soften, melt or dissolve in the cavity fluids.

In general, for suppositories, traditional binders and carriers may include, for example, polyalkylene glycols or triglycerides; such suppositories may be formed from mixtures containing the active ingredient in the range of 0.5% to 10%, preferably 1%-2%.

Vaginal suppositories or pessaries are usually globular or oviform and weighing about 5 g each. Vaginal medications are available in a variety of physical forms, e.g., creams, gels or liquids, which depart from the classical concept of suppositories. Vaginal tablets, however, do meet the definition, and represent convenience both of administration and manufacture.

Oral formulations include such normally employed excipients as, for example, pharmaceutical grades of mannitol, lactose, starch, mangesium stearate, sodium saccharine, cellulose, magnesium carbonate and the like. These compositions take the form of solutions, suspensions, tablets, pills, capsules, sustained release formulations or powders.

In certain defined embodiments, oral pharmaceutical compositions will comprise an inert diluent or assimible edible carrier, or they may be enclosed in hard or soft shell gelatin capsule, or they may be compressed into tablets, or they may be incorporated directly with the food of the diet. For oral therapeutic administration, the active compounds may be incorporated with excipients and used in the form of ingestible tablets, buccal tables, troches, capsules, elixirs, suspensions, syrups, wafers, and the like. Such compositions and preparations should contain at least 0.1 % of active compound. The percentage of the compositions and preparations may, of course, be varied and may conveniently be between about 2 to about 75% of the weight of the unit, or preferably between 25-60%. The amount of active compounds in such therapeutically useful composition is such that a suitable dosage will be obtained.

The tablets, troches, pills, capsules and the like may also contain the following: a binder, as gum tragacanth, acacia, cornstarch, or gelatin; excipients, such as dicalcium phosphate; a disintegrating agent, such as corn starch, potato starch, alginic acid an the like; a lubricant, such as magnesium stearate; and a sweetening agent, such as sucrose, lactose or saccharin may be added or a flavoring agent, such as peppermint, oil of wintergreen, or cherry flavoring. When the dosage unit form is a capsule, it may contain, in addition to material of the above type, a liquid carrier. Various other materials may be present as coatings or to otherwise modify the physical form of the dosage unit. For instance, tablets, pills, or capsules may be coated with shellac, sugar or both. A syrup of elixir may contain the active compounds sucrose as a sweetening agent methyl and propylparabens as preservatives, a dye and flavoring, such as cherry or orange flavor.

### Transdermal Delivery

Although essentially any method of delivery of a compound that prevents or decreases the interaction of a NK-T cell receptor and its respective ligand may be used, in embodiments of treating disorders of the skin it is preferred that the compound is delivered transdermally.

During recent years much attention has been paid to the development of transdermal delivery systems as a means of mitigating many of the drawbacks associated with the parenteral or oral route of administration. (Sloan K B, Adv. Drug Delivery Rev. (1989), 67-101). A prerequisite for the development of a transdermal delivery system of a compound that prevents or decreases the interaction of a NK-T cell receptor and its respective ligand is, however, that the compound that prevents or decreases the interaction of a NK-T cell receptor and its respective ligand is capable of penetrating the skin at a sufficiently high rate and is not metabolized during the percutaneous absorption.

It is an object of the present invention to provide a transdermal delivery system for a compound that prevents or decreases the interaction of a NK-T cell receptor and its respective ligand. Transdermal medicaments are well known to those of skill in the art (see for example U.S. Patent No. 5,908,846; U.S. Patent No. 5,904,930; U.S. Patent No. 5,902,603; U.S. Patent No. 5,902,602; U.S. Patent No. 5,891,461; U.S. Patent No. 5,883,115; U.S. Patent No. 5,876,746; U.S. Patent No. 5,858,393). In specific embodiments, the transdermal medicament may be a patch, a gel matrix, a cream, a colloid, an ointment, a lotion or any other suitable form of topical transdermal delivery.

In particularly preferred embodiments, the present invention provides a transdermal patch comprising a reservoir containing a compound that prevents or decreases the interaction of a NK-T cell receptor and its respective ligand in a nonaqueous matrix. The patch may be placed directly on the lesion.

Methods and compositions for producing medicaments for transdermal delivery are well known to those of skill in the art. The patents listed above have been incorporated herein by reference to provide guidance for making such transdermal medicaments.

The actual administration or use of the transdermal compositions according to the present invention can be in any conventional form and may follow any of the methods generally known to the art. For instance, a compound that prevents or decreases the interaction of a NK-T cell receptor and its respective ligand can be used in association with any pharmaceutical dosage form such as, for example, but not limited thereto, any solution, ointment, lotion, paste, jelly, gel, cream, spray or aerosol generally known to the art. As such, a compound that prevents or decreases the interaction of a NK-T cell receptor and its respective ligand in association with the pharmaceutical dosage form can be used directly as a topical composition or used in combination with an additional drug delivery device, for example, but not limited thereto, patches, gauze, compresses, or the like, again, as generally known in the art. The dosage forms may contain any type of absorption or permeation enhancers such as fatty acids, fatty acid esters and fatty alcohols or any other non-toxic compounds which are known to increase skin permeability. In particular, the transdermal compositions, can be administered in the form of a h7patch wherein, a compound that prevents or decreases the interaction of a NK-T cell receptor and its respective ligand is present in a polymeric matrix or in a reservoir system combined with a polymeric rate controlling membrane.

"Permeation enhancer" or "absorption enhancer" as used herein relates to any agent that increases the permeability of skin to a compound that prevents or decreases the interaction of a NK-T cell receptor and its respective ligand i.e., so as to increase the rate at which a compound that prevents or decreases the interaction of a NK-T cell receptor and its respective ligand penetrates through the skin and enters the bloodstream. The enhanced permeation effected through the use of such enhancers can be observed by measuring the rate of diffusion of drug through animal or human skin using a diffusion cell apparatus. Such devices are well known to those of skill in the art (see for example U.S. Patent No. 5,906,830).

U.S. Patent 5,906,830 describes methods for manufacturing transdermal drug delivery systems containing supersaturated drug reservoirs, such that higher drug fluxes are obtained. These methods involve heating the drug reservoir components to a predetermined temperature. Generally, this temperature is higher than the depressed melting temperature of the polymer-drug admixture which will serve as the drug reservoir. It is contemplated that the methods described therein will be useful for producing the transdermal delivery medicaments of the present invention.

U.S. Patent No. 4,409,206 relates to a method for preparing transdermal drug delivery systems containing the active substance in an amorphous form. Initially, a polyacrylate film is prepared by solvent casting. A drug solution or suspension is then applied to the film and the solvent is removed by evaporation. U.S. Patent No. 4,746,509 describes transdermal medicaments with the active ingredient dispersed in a drug reservoir in crystalline and/or solubilized form. Another method that may be useful in the present invention is described in U.S. Patent No. 4,832,953 in which the drug delivery systems containing liquid drugs capable of forming crystalline hydrates are formed. U.S. Patent No. 4,883,669 describes a transdermal drug delivery system in which the drug is microdispersed in a polymeric matrix disc layer which serves as the drug reservoir. U.S. Patent No. 5,332,576 describes preparation of compositions for topical application, in which the drug is added to certain components, not including the bioadhesive carrier, and then heated at a temperature in the range of about 70°C to 90°C until all of the drug is dissolved. After the solution is cooled, the bioadhesive is added and the composition is applied to a backing material. It is contemplated that such methods of producing a transdermal delivery system may be used herein.

In addition, transdermal delivery forms may further include diffusional systems and dissolution systems. In diffusional systems, the release rate of drug is further effected by its rate of diffusion through a water-insoluble polymer. There are generally two types of diffusional devices, reservoir devices in which a core of drug is surrounded by polymeric membrane; and matrix devices in which dissolved or dispersed drug is distributed substantially uniformly and throughout an inert polymeric matrix. In actual practice, many systems that utilize diffusion may also rely to some extent on dissolution to determine the release rate.

Common practices utilized in developing transdermal delivery devices with reservoir systems include microencapsulation of drug particles (e.g., see U.S. Patent 5,814,599). Frequently, particles coated by microencapsulation form a system where the drug is contained in the coating film as well as in the core of the microcapsule. As such, in transdermal delivery, such a microcapsule may be applied as a microemulsion or be coated onto a patch-type delivery system. Drug release typically includes a combination of dissolution and diffusion with dissolution being the process that controls the release rate. Common material used as the membrane barrier coat, alone or in combination, include but are not limited to, hardened gelatin, methyl and ethyl-cellulose, polyhydroxymethacrylate, polyvinylacetate, and various waxes.

The most common method of microencapsulation is coacervation, which involves addition of a hydrophilic substance to a colloidal dispersion. The hydrophilic substance, which operates as the coating material, is selected from a wide variety of natural and synthetic polymers including shellacs, waxes, starches, cellulose acetate, phthalate or butyrate, polyvinyl-pyrrolidone, and polyvinyl chloride. After the coating material dissolves, the drug inside the microencapsule is immediately available for dissolution and absorption. Drug release, therefore, can be controlled by adjusting the thickness and dissolution rate of the coat. For example, the thickness can be varied from less than one µm to 200 µm by changing the amount of coating material from about 3 to about 30 percent by weight of the total weight. By employing different thicknesses, typically three of four, the active agent will be released at different, predetermined times to afford a delayed release affect.

In matrix systems, three major types of materials are frequently used in the preparation of the matrix systems which include insoluble plastics, hydrophilic polymers, and fatty compounds. Plastic matrices which have been employed include methyl acrylate-methyl methacrylate, polyvinyl chloride and polyethylene. Hydrophilic polymers include methyl cellulose, hydroxypropylcellulose, hydroxypropyl-ethylcellulose, and its derivatives and sodium carboxy-methylcellulose. Fatty compounds include various waxes such as camauba wax, and glyceryl tristearate. Preparation of these matrix systems are by methods well known to those skilled in the art. These methods of preparation generally comprise mixing the drug with the matrix material and compressing the mixture into a suitable pharmaceutical layer. With wax matrixes, the drug is generally dispersed in molten wax, which is then congealed, granulated and compressed into cores.

Approaches to further reducing the dissolution rate include, for example, coating the drug with a slowly dissolving material, or incorporating the drug into a formulation with a slowly dissolving carrier. Encapsulated dissolution systems are prepared either by coating particles or granules of drug with varying thicknesses of slowly soluble polymers or by microencapsulation.

Matrix dissolution systems are prepared by compressing the drug with a slowly dissolving polymer carrier into formulation suitable for transdermal delivery such as a gel matrix, a cream, a colloid, an ointment, a lotion or any other suitable form of topical transdermal delivery. Generally there are two methods for preparing drug-polymer particles, congealing and aqueous dispersion methods. In the congealing method, the drug is mixed with a polymer or wax material and either cooled or cooled and screened or spray-congealed. In the aqueous dispersion method, the drug-polymer mixture is simply sprayed or placed in water and the resulting particles are collected.

Osmotic systems are also available where osmotic pressure is employed as the driving force to afford release of a drug. Such systems generally consist of a core of drug surrounded by a semi-permeable membrane containing one or more orifices. The membrane allows diffusion of water into the core, but does not allow release of the drug except through the orifices. Examples of materials used as the semi-permeable membrane include polyvinyl alcohol, polyurethane, cellulose acetate, ethylcellulose, and polyvinyl chloride.

In a particularly preferred embodiment, it is contemplated that topical dosage forms are in the form of a compound that prevents or decreases the interaction of a NK-T cell receptor and its respective ligand-containing patch. It is contemplated that such a patch comprises a matrix type or reservoir type patch system containing a compound that prevents or decreases the interaction of a NK-T cell receptor and its respective ligand in combination with a penetration enhancing delivery device/process such as iontophoresis, electroporation or ultrasound. Reservoir type patch systems and iontophoresis are both well known systems for transdermal delivery. Accordingly, it is contemplated that the use of these transdermal delivery systems afford the appropriate permeability coefficients and fluxes of a compound that prevents or decreases the interaction of a NK-T cell receptor and its respective ligand through a predetermined area of mammalian skin tissue. These coefficients and fluxes are preferably established as being sufficient in magnitude to be practical for producing time-sustained dosage rates consistent for the therapeutic effects of a compound that prevents or decreases the interaction of a NK-T cell receptor and its respective ligand over prolonged periods of time.

By "predetermined area of mammalian skin tissue" is intended to refer to a defined area of intact unbroken living skin or mucosal tissue. That area will usually be in the range of about 5 cm² to about 100 cm², more usually in the range of about 20 cm² to about 60 cm². However, it will be appreciated by those skilled in the art of transdermal drug delivery that the area of skin or mucosal tissue through which drug is administered may vary significantly, depending on patch configuration, dose, and the like.

A patch device generally comprises a laminated composite of a drug reservoir, a backing layer which serves as the upper surface of the device during use and is substantially impermeable to the drug, and a release liner to protect the basal surface of the device prior to use. Optionally, a contact adhesive layer or a peripheral ring of contact adhesive may be provided on the basal surface of the device to enable adhesion of the device to the skin during drug delivery.

The backing layer functions as the primary structural element of the device and provides the device with much of its flexibility, drape and, preferably, occlusivity. The material used for the backing layer should be inert and incapable of absorbing drug, enhancer or other components of a composition comprising a compound that prevents or decreases the interaction of a NK-T cell receptor and its respective ligand contained within the device. The backing is preferably made of one or more sheets or films of a flexible elastomeric material that serves as a protective covering to prevent loss of drug and/or vehicle via transmission through the upper surface of the device, and will preferably impart a degree of occlusivity to the device, such that the area of the skin covered on application becomes hydrated. The material used for the backing layer should permit the device to follow the contours of the skin and be worn comfortably on areas of skin such as at joints or other points of flexure, that are normally subjected to mechanical strain with little or no likelihood of the device disengaging from the skin due to differences in the flexibility or resiliency of the skin and the device. Examples of materials useful for the backing layer are polyesters, polyethylene, polypropylene, polyurethanes and polyether amides. The layer is preferably in the range of about 15 µM to about 250 µM in thickness, and may, if desired, be pigmented, metallized, or provided with a matte finish suitable for writing.

The reservoir layer component of the transdermal patch may double as the means for containing a compound that prevents or decreases the interaction of a NK-T cell receptor and its respective ligand-containing formulation and as an adhesive for securing the device to the skin during use. That is, as the release liner is removed prior to application of the device to the skin, the reservoir layer serves as the basal surface of the device which adheres to the skin.

Suitable polymeric materials for the drug reservoir include pressure-sensitive adhesives which are physically and chemically compatible with the drug to be administered, and the carriers and vehicles employed. Such adhesives include, for example, polysiloxanes, polyisobutylenes, polyacrylates, polyurethanes, plasticized ethylene-vinyl acetate copolymers, low molecular weight polyether amide block polymers (e.g., PEBAX), tacky rubbers such as polyisobutene, polystyrene-isoprene copolymers, polystyrene-butadiene copolymers, and mixtures thereof. In addition, other adhesive materials for use as reservoir layer include acrylates, silicones and polyisobutylenes, combinations of acetate-acrylate copolymers (such as may be obtained under the trademarks GELVA^{™} 737 and GELVA^{™} 788 from Monsanto Chemical Co.) with a water soluble, water-absorptive polymer such as polyvinyl alcohol, gelatin, polyacrylic acid, sodium polyacrylate, methylcellulose, carboxymethylcellulose, polyvinylpyrrolidone, gum acacia, gum tragacanth, carrageenan and guar gum, particularly polyvinylpyrrolidone.

The reservoir layer is comprised of an adhesive material as described above, and will generally range in thickness from about 10 to about 300 µM, preferably approximating 75 µM. Alternatively, the adhesive layer may form a separate and distinct portion of the device, for example, the adhesive portion of the device may comprise an annulus around the reservoir layer, this annulus serving to ensure that the patch device adheres to the skin. Similarly, the reservoir area and the adhesive area may be sandwiched between each other as strips or annular portions, for example a patch is contemplated in which there are stripes of drug reservoir separated by stripes of adhesive.

The release liner is a disposable element which serves only to protect the device prior to application. Typically, the release liner is formed from a material impermeable to the drug, vehicle and adhesive, and which is easily stripped from the contact adhesive. Release liners are typically treated with silicone or fluorocarbons.

Any of these transdermal drug delivery devices may also be provided with a release rate controlling membrane to assist in controlling the flux of drug and/or vehicle from the device. Such a membrane will be present in a drug delivery device beneath and typically immediately adjacent to the drug reservoir, and generally between the drug reservoir itself and an adhesive layer which affixes the device to the skin. Representative materials useful for forming rate-controlling membranes include polyolefins such as polyethylene and polypropylene, polyamides, polyesters, ethylene-ethacrylate copolymer, ethylene-vinyl acetate copolymer, ethylene-vinyl methylacetate copolymer, ethylene-vinyl ethylacetate copolymer, ethylene-vinyl propylacetate copolymer, polyisoprene, polyacrylonitrile, ethylene-propylene copolymer, and the like.

### Liposomes and Nanocapsules

In certain embodiments, the use of liposomes and/or nanoparticles is contemplated for the introduction of antibodies, polypeptides or agents, or gene therapy vectors, including both wild-type and antisense vectors, into host cells. The formation and use of liposomes is generally known to those of skill in the art, and is also described below.

Nanocapsules can generally entrap compounds in a stable and reproducible way. To avoid side effects due to intracellular polymeric overloading, such ultrafine particles sized around 0.1 µm) should be designed using polymers able to be degraded *in vivo.* Biodegradable polyalkyl-cyanoacrylate nanoparticles that meet these requirements are contemplated for use in the present invention, and such particles may be are easily made.

Liposomes are formed from phospholipids that are dispersed in an aqueous medium and spontaneously from multilamellar concentric bilayer vesicles (also termed multilamellar vesicles (MLVs). MLVs generally have diameters of from 25 nm to 4 µm. Sonication of MLVs results in the formation of small unilamellar vesicles (SUVs) with diameters in the range of 200 to 500 A, containing an aqueous solution in the core.

The following information may also be utilized in generating liposomal formulations. Phospholipids can form a variety of structures other than liposomes when dispersed in water, depending on the molar ratio of lipid to water. At low ratios the liposome is the preferred structure. The physical characterisics of liposomes depend on pH, ionic strength and the presence of divalent cations. Liposomes can show low permeability to ionic and polar substances, but at elevated temperatures undergo a phase transition which markedly alters their permeability. Th phase transition involves a change from a closely packed, ordered structure, known as the gel state, to a loosely packed, less-ordered structure, known as the fluid state. This occurs at a characteristic phase-transition temperature and results in an increase in permeability to ions, sugars and drugs.

Liposomes interact with cells via four different mechanisms: Endocytosis by phagocytic cells of the reticuloendothelial system such as macrophages and neutrophils; adsoprtion to the cell surface, either by nonspecific weak hydrophobic or electrostatic forces, or by specific interactions with cell-surface components; fusion with the plasma cell membrane by insertion of the lipid bilayer of the liposome into the plasma membrane, with simultaneous release of liposomal contents into the cytoplasm; and by transfer of liposomal lipids to cellular or subcellular membranes, or *vice versa,* without any association of the liposome contents. Varying the liposome formulation can alter which mechanism is operative, although more than one may operate at the same time.

### Example 1: CD161-CD1d Interaction in Psoriasis

Having established the presence and functional role for CD94/CD158a/CD158b+NK-T cells and classical MHC class I positive antigen presenting cells in psoriasis, this example demonstrates that the CD161 molecule, which is also a c-lectin like receptor, is also present in psoriasis and activated by its known ligand-CD1d (3).

### Human Tissue Samples

3-4 mm punch biopsies of normal adult human skin (NN skin; n=6), symptomless skin from patients with psoriasis elsewhere (PN skin; n=8), active, untreated psoriatic plaques (PP skin; n=8) were obtained after local anesthesia and informed consent. Similarly, punch biopsies were also obtained from volunteers in which epicutaneous poison-ivy antigen was applied in previously sensitized individuals (n=3), as well as from patients with Bowen's disease (n=5), squamous cell carcinoma (n=5), or basal cell carcinoma (n=8). In one patient with psoriasis, a biopsy ofPN skin 72 hours after leaving the barrier function perturbed by repeated tape stripping was also examined. Thymic tissue was obtained from children undergoing elective cardiothoracic surgery. All tissue procurement was obtained with approval by institutional review boards. Tissue samples were snap frozen in isopentane chilled in liquid nitrogen, and stored at -80° C until cryostat sections (5 micron thick) were cut and placed on glass slides. Some of these biopsies were obtained during the course of an earlier investigation (24).

### SCID Mouse-Human Skin Model of Psoriasis

The engraftment of normal human skin onto SCID mice followed by injection of activated immunocytes from peripheral blood of a psoriatic patient were previously described (10). Cryostat sections of the psoriatic plaque that was created involved both murine and human skin components as described (10).

### Tissue Culture

Multipassaged normal human keratinocytes and an immortalized human keratinocyte cell line (HaCaT) were grown in a low-calcium, serum-free medium (KGM, Clonetics Corp., San Diego, CA), as previously described (25,26).

### Immunohistochemical Staining

Cryostat sections were fixed using cold acetone followed by use of an avidi-biotin peroxidase staining procedure as previously described (27). Briefly, primary antibodies (10 µg/ml) were incubated for 30 mins at room temperature followed by subsequent steps as per the manufacturer's instructions (Vectastain; Vector Laboratories; Burlingame, CA). Positive detection was accomplished using 3 amino - 4 ethylcarbazole as the chromagen producing a red reaction product with 1% hematoxylin as the counterstain (27). Primary murine antibodies included anti-CD1d (Clone NOR3.2 IgG, Biosource), anti-Vβ11 (IgG, Becton-Dickinson, Mountainview, CA), and anti-Vα24; anti-CD 161 and anti-CD94 were purchased from Coulter Corp. (Hialeah, FL). Detectin of murine CD1d was accomplished using rat anti-mouse CD1d antibody (Pharmingen, Sandiego, CA) For staining of cultured keratinocytes, 8 well Lab-Tek slides (Nunc Inc, Napervillle, IL) were used. Semi-confluent keratinocyte cultures were exposed to IFN-γ (10³ U/ml; Genzyme, Cambridge, MA), phorbol ester (TPA, 10 nM; Sigma Chemical Co., St. Louis, MO), tumor necrosis factor-alpha (TNF-α; 10³ U/ml; Genzyme), transforming growth factor-beta (TGF-β; 10 ng/ml; Genzyme), IL-1β (200 pg/ml; Genzyme) or IFN-γ plus TPA; or IFN-γ plus TNF-α, for either 24 or 48 hours.

### RESULTS

### I. Expression of CD1d in thymus and normal human skin and symptomless (PN) skin.

Since CD1d was previously localized to thymic epithelium using a different antibody, the currently used mAb (i.e. NOR3.2) was first tested on thymic tissue. Strong epithelial surface expression on Hassel's corpuscles was noted producing concentric rings of positive immunoreactivity, and there was also staining of scattered cortical thymocytes, dendritic cells and endothelial cells. Next, normal skin biopsies were examined and CD 1d was consistently present in the 3-4 outermost layers of keratinocytes as distinct cytoplasmic membrane staining. Occasional staining of the basal cell layer or mid-layers of keratinocytes in the stratum spinosum was seen in some normal skin biopsies. Thus, most of the keratinocytes in the stratum granulosum were CD1d positive which extended up to the lipid-rich-containing stratum corneum.

There was no apparent CD1d expression noted on Langerhans cells, but weak, focal CD1d was present on dermal dendritic cells and endothelial cells. A similar immunohistochemical staining pattern emphasizing upper level keratinocytes CD1d expression was also present in PN skin as well as dermal dendritic cells and endothelial cells. For both normal and PN skin, CD1d was also present focally on eccrine duct epithelium and acrosyringium. The dermal papillae and hair matrix were CD 1d negative, but the inner root sheath epithelium was diffusely positive with some anagen. Follicles displaying outer root sheath epithelium and sebocytes being CD1d positive. No CD1d was discerned on dermal mast cells, fibroblasts, or in subcutaneous tissue sites.

### II. Expression of CD1d in symptomless skin; psoriatic plaques; allergic contact dermatitis, and after repeated tape-stripping.

In contrast to normal and symptomless skin, chronic psoriatic plaques had more extensive keratinocyte CD1d expression. Since the granular cell layer is generally absent in a psoriatic lesion (28), CD1d expression began just beneath the large amount of lipid-rich scale, and extended to include 8-12 layers beneath the stratum corneum to suprabasilar layers. CD1d was primarily confined to plasma membrane producing a broad "chicken-wire" appearance to the plaques. There was focal CD1d expression in the basal cell layer in several of the plaques; as well as more diffuse and intense CD I d on dermal dendritic cells and endothelial cells. Within 72 hours of barrier perturbation accomplished by repeated tape stripping, symptomless skin acquired diffuse epidermal CD1d expression underlying appearance of parakeratotic scale.

Since early lesions of psoriasis can resemble evolving allergic contact dermatitis reactions, skin biopsies obtained from sensitized individuals exposed epicutaneously to the hydrophobic wax-containing oil of the poison-ivy leaf (i.e. urushiol) were examined (24). 8 hours after exposure, no changes in CD1d expression were noted, but as early as 24 hours following exposure, biopsies revealed increased CD1d expression by epidermal keratinocytes, and at 48 hours as mononuclear cells appeared in the upper dermis and epidermis, keratinocytes in the mid and superficial epidermal layers were CD1d positive.

### I. Expression of CD1d in basal cell carcinoma, Bowen's disease and squamous cell carcinoma.

Infiltrating tumor cells from basal cell carcinomas were generally devoid of CD 1d expression although in some specimens, tumor cells immediately adjacent to zones of apoptosis were CD1d positive. In squamous cell carcinoma-in-situ (Bowen's disease); atypical intraepidermal keratinocytes expressing CD1d were identified with some variation amongst the cases. In some cases, CD1d expression was present in atypical cells focally in the basal cell layer and mid-epidermis, whereas in other cases there was more diffuse mid layer to upper layer CD1d expression by the atypical keratinocytes.

In the invasive squamous cell cacinomas, numerous clusters of malignant cells were CD1d positive including the lobular acantholytic tumor cells. There was some variation from case to case in the overall percentage of malignant cells expressing CD1d ranging from approximately 20% of the infiltrating tumor cells, to over 80% of the tumor cells possessing cytoplasmic membrane CD1 positivity. CD1d was detected on both well differentiated as well as poorly differentiated squamous carcinoma tumor cells.

### IV. Expression of CD161, CD94, Vα24 and Vβ11 in normal and diseased skin samples.

While there were rare CD161, Vα24, or Vβ11 positive lymphocytes in normal and symptomless skin, in some cases in which there was increased CD1d expression by benign and malignant keratinocytes, numerous intraepidermal lymphocytes expressing Vα24 or Vβ11 were occasionally observed. Observed was one large invasive island of squamous cell carcinoma with numerous Vα24 positive lymphocytes. Of note was the absence of Vα24 positive lymphocytes in the surrounding stroma, and their activated appearance in the tumor island with numerous cytoplasmic extensions or spikes partially enveloping the malignant epithelial cells. Compared to the sporadic presence of Vα24 or Vβ11 positive lymphocytes, there was more consistent and more frequent CD94 and CD 161 positive NK-T cells in skin biopsies with prominent CD1d expression.

Of all the specimens examined, the most striking immunohistochemical finding was the co-presence of CD 161 positive NK-T cells and CD1d positive keratinocytes - particularly apparent in psoriasis. Only occasional CD 161 positive NK-T cells were seen in squamous cell carcinoma. In one psoriatic plaque, there was also a prominent collection of CD94 positive NK-T cells in the hyperplastic epidermis.

### V. Modulation of CD1d in cultured human keratinocytes.

Normal multi-passaged human keratinocytes or HaCaT cells grown using a low-calcium, serum-free medium constitutively expressed CD1d in a focal pattern. However, following 24 hours of exposure to various cytokines such as IFN-γ, TNF-α, IL-1β, enhanced CD1d expression by keratinocytes decorating their plasma membranes was observed. However, exposure of keratinocytes to TPA or TGF-β did not enhance CD1d expression. Combinations of IFN-γ plus TNF-α or IFN-γ plus TPA also resulted in increased CD1d expression. Both immortalized HaCaT cells (passages 134-135) and primary cultured keratinocytes produced similar immunohistochemical staining profiles in response to the aforementioned stimuli.

### VI. SCID-Human Skin Xenograft

The psoriatic lesion created by intraepidermal injection into engrafted normal skin of pathogenic T cells revealed CD1d expression on both the hyperplastic human and murine epidermis which was infiltrated by human CD161 positive NK-T cells in both human and murine components of the graft (3).

The rapid influx of CD161+ NK-T cells and up-regulation of keratinocyte CD1d expression by both human and murine epidermal cells immediately prior to lesion formation point to the involvement of these novel ligand/receptor interactions in creating psoriasis both in normal human and murine skin. Besides CD 161 and CD1d interactions that can occur between mice and men, no other ligand/receptor recognition events are known (29).

### DISCUSSION

Despite earlier reports which suggested relative confinement of CD1d to intestinal epithelial cells (30-32), there is CD I d expression in normal human skin, as well as in several skin disorders including psoriasis. In normal human skin, CD1d was generally restricted to the outermost keratinocyte layers in the stratum granulosum just beneath the lipid containing stratum corneum. It appeared that the plasma membrane staining for CD1d was juxtaposed directly with the stratum corneum, although CD1d could also be seen in some specimens to be present in the basal cell layer as well. In addition to epidermal keratinocytes, CD1d was detected on upper dermal dendritic cells, endothelium, eccrine ducts, acrosyringium and the pilosebaceous unit except for the dermal papilae and hair matrix cells.

CD1d expression could be rapidly modulated in-vivo as exemplified by the response of normal skin 24 hours of induction of allergic contact dermatitis involving the poison ivy antigen, or after repeated tape stripping of the symptomless skin. Similarly, in-vitro exposing cultured keratinocytes to various cytokines, keratinocytes were induced to strongly and diffusely express CD1d. Thus, like class I MHC antigens and class II MHC antigens, HLA-DR, HLA-DQ, as well as intercellular adhesion molecule-1 (ICAM-1), keratinocytes can also be induced to express CD1d (33).

In psoriatic plaques CD1d expression was increased compared to normal and symptomless skin and also extended to the outermost keratinocytes immediately beneath the parakeratotic layer juxtaposed to the stratum-corneum. Given this intimate anatomical configuration, it is possible for various types of glycolipids in the psoriatic scale to be directly exposed to the abundant keratinocyte cell surface CD1d. Moreover, given the large hydrophobic binding pockets in CD1d, the presence of CD1d on the outer layers of epidermis in psoriatic plaques opens up the possibility thai various glycolipids present in the stratum corneum could play a role in triggering a response by NK-T cells or other T cell subsets capable of recognizing such glycolipids in the context of CD1d.

During epidermal differentiation keratinocytes produce different amounts and types of various glycolipids including glucosylceramides (34). Alterations in these glycolipids in the stratum corneum can significantly impact the barrier function of skin. Others have focused on the direct proliferative effect of glucosylceramides on epidermal keratinocytes (35). However, it is also clear that barrier perturbation can initiate cytokine -cascades impacting inflammatory and mononuclear cell activation (36,37). The rapid and diffuse expression seen in the symptomless skin following repeated tape stripping demonstrates the highly responsive inductive capability of keratinocytes with respect to CD1d expression (37). Furthermore, a reciprocal interaction appears likely since T cells can also influence barrier function (38).

As regards psoriasis, abnormalities in barrier function have been well documented (39, 40) with both quantitative and qualitative changes including alterations in glycolipids, that may not be similarly present in other scaly dermatitis such as atopic dermatitis (41). Thus, a viscious cycle is created in that pathogenic NK-T cells initiate barrier abnormality which in turn would generate glycolipids that could be presented by keratinocyte CD1d and further activate CD161 NK-T cells in psoriasis, allergic contact dermatitis, and other disorders.

Many psoriatic plaques show clinical improvement by simply occluding the skin to alter the local barrier function (42). This phenomenon could be explained if pro-T cell activating glycolipids were displaced from their CD1d binding groove by non-stimulatory types of lipids.

That CD 161 positive NK-T cells can interact with CD1d positive keratinocytes is derived from a surprising result in which the injection of pathogenic blood-derived immunocytes from a psoriatic patient injected into engrafted normal skin from a non-related donor induced psoriasis on both the human as well as murine skin. Observed were CD 161 positive human NK-T cells infiltrating the murine CD1d positive hyperplastic epidermis. Thus, due to the highly conserved structure of CD1d (2), human CD161 + NK-T cells produce psoriasis via this interaction.

As regards the triggering of NK-T cells, it must also be mentioned that these interesting T cells bear both T cell receptors (TCRs) as well as NKRs. Thus, they may be under the dual influence of a peptide-type antigen that could trigger T cell activation via the TCR, as well as a non-peptide, glycolipid type antigen via the NKR. There is ample precedent for such synergistic interactions (43), and, many new intriguing approaches to our understanding of psoriasis can now be pursued. Since there was relatively infrequent Vα24 and β11 positive cells in psoriasis, a different structurally conserved TCR may be the immunodominant TCR involved in interacting with CDId as recently observed in murine epidermis (44).

CD1d expression was not confined to allergic contact dermatitis or psoriasis, but was present in pre-malignant and malignant keratinocytes. While there was rather diffuse CD1d expression by keratinocytes in Bowen's disease and invasive squamous cell carcinomas, the infiltrating tumor cells in basal cell carcinoma had more focal CD1d expression.

Besides keratinocytes being able to initiate, perpetuate, and terminate immune reactions involving conventional T cell responses to nominal antigens and superantigens, CD1d expression may also imbue the keratinocyte with the capacity to interact with NK-T cells.

### Example 2: CD161+ NK-T Cells Are Pathogenic

Example 1 establishes that CD161-CD1d interaction plays a role in psoriasis. However, it could not be determined whether the NK-T cells were functioning directly (pathogenic) or indirectly (immunoregulatory) to cause psoriasis. This example demonstrates the direct (pathogenic) role of NK-T cells in psoriasis.

Immunocytes recovered from peripheral blood of a psoriatic patient were exposed to bacterial superantigens and IL-2. After several weeks, a line was produced. FACS analysis revealed that the cells were predominantly CD3+ T cells that were all CD4+ T cells. The majority of these cells expressed CD161. Thus, the cells were predominantly NK-T cells. These CD161+ NK-T cells were pathogenic because when the cells were injected into engrafted PN skin, a psoriatic plaque was produced.

The NK-T cell line was capable of interacting with CD1d expressing keratinocytes. Cultured keratinocytes were induced to express CD1d with IFN-gamma. Under these conditions, the cells were mixed with the NK-T cell line. The INF-gamma treated keratinocytes caused proliferation of the NK-T cells. Controls containing the NK-T cells alone or keratinocytes not treated with IFN-gamma failed to induce proliferation of the NK-T cells.

The proliferation of the NK-T cells was dependent on CD1d expression on the keratinocytes. When IFN-gamma and the NK-T cell line were incubated together in the presence of anti-CD1d blocking antibody, proliferation of the NK-T cells was greatly reduced as compared to controls lacking the antibody.

Besides proliferation, the NK-T cells were also shown to produce cytokines (IFN-gamma and IL-13) after recognition of CD1d on the keratinocytes. ELISA assays showed that high levels of IFN-gamma were produced by the NK-T cells incubated with IFN-gamma treated keratinocytes as compared to NK-T cells alone or NK-T cells with untreated keratinocytes (both of wish essentially lacked any IFN-gamma production). As with proliferation, IFN-gamma production by the NK-T cells was inhibited by coincubation with anti-CD1d antibody.

Because IFN-gamma is a key cytokine in triggering psoriasis (1,2), this *in vitro* system provides an excellent model for testing the ability of various compounds to block the activating interaction of NK-T cells and keratinocytes. The ability of these compounds to block CD 161 and CD1d interaction and thereby prevent the ability of the NK-T cells to induce psoriasis *in vivo* may also be determined as described in Example 1.

### References

1. Nickoloff BJ: Cytokine network in psoriasis. Arch Dermatol, 127: 871-884,1991.
2. Nickoloff, BJ, "The immunologic and genetic basis of psoriasis," *Arch Derm, 1999.*
3. Nickoloff BJ, *et al.,* "Response of murine and normal human skin to injection of allogeneic blood-derived psoriatic immunocytes," *Arch Derm*, 135:546-552, 1999.
4. Nickoloff *et al.,* "Injection of CD4+ T cells into pre-psoriatic skin induces psoriasis," *Am J Pathol,* 1999.
5. Takeda K, Dennert G: The development of autoimmunity in C57Bl/6 LPR mice correlates with the disappearance of natural killer type I-positive cells: evidence for their suppressive action on bone marrow stem cell proliferation, B cell immunoglobulin secretion, and autoimmune symptoms. J Exp Med 177: 155-162, 1993.
6. Yoshimoto T, Bendelac A. Watson C, Hu-Li J. Paul WE: Role of NK-1. 1+ T cells in a TH2 response and in immunoglobulin E production. Science 270: 1845-1847, 1995.
7. Sumida T, Sakemoto A, Murata H, Makino Y, Takahashi H, Yoshida S, Neshioka K, Iwarnoto I, Taniguchi M: Selective reduction of T cells bearing invariant Vα24JαQ antigen receptor in patients with systemic sclerosis. J Exp Med 182: 1163-1168, 1995.
8. Cui J, Shin T, Kawano T, Satoth, Kondo E, Toura I, Kaneko Y, Koseki H, Kanno M, Taniguchi M: Requirement for Vα 14 NK T cells in IL-12-mediated rejection of tumors. Science 278: 1623-1626, 1997.
9. Nickoloff *et al.*, "Severe combined immunodeficiency mouse and human psoriatic skin chimeras. Validation of a new animal model," *Am J Pathol.*, 146:580-588, 1995.
10. Wrone-Smith T, Nickoloff BJ: Dermal injection of immunocytes induces psoriasis. J Clin Invest 98: 1878-1887, 1996.
11. Nickoloff BJ, Wrone-Smith T: Autoantigens, superantigens and pathogenic T cells in psoriasis. J Invest Dermatol 110: 459-460, 1998.
12. Long EU, Colonna M, Lanier LL: Inhibitory MHC class I receptors on NK and T cells: a standard nomenclature. Immunol Today 17:100, 1996.
13. Mandelboim O, Reyburn HT, Vales-Gomez M, Pazmany L, Colonna M, Borsellino G, Strominger JL: Protection from lysis by natural killer cells of groups 1 and 2 specificity is mediated by residue 80 in human histocompatibility leukocyte antigen C alleles and also occurs with empty histocompatibility complex molecules. J Exp Med 184: 913-922, 1996.
14. Asahina A, Akazaki S, Nakagawa H, Kuwata S, Tokunaga K, Ishibashi Y, Juji T: Specific nucleotide sequence of HLA-C is strongly associated with psoriasis antigens. J Invest Dermatol 97: 254-258, 1991.
15. Davis *et al.,* "The transmembrane sequence of human histocompatibility leukocyte antigen(HLA)-C as a determinant in inhibition of a subset of natural killer cells," *J Exp Med,* 189:1265-1274, 1999.
16. Capon *et al.*, "Searching for psoriasis susceptibility gene in Italy. Genome scan and evidence for a new locus on chromosome 1," *J Invest Dermatol,* 112:32-35, 1999.
17. Perez-Villar JJ, Melero I, Navarro F, Carretero M, Bellow T, Llano M, Colonna M, Geraghty DE, Lopez-Botet M: The CD94/NKG2-A inhibitory receptor complex is involved in natural killer cell-mediated recognition of cells expressing HLA-G1. J Immunol 158:5736-5743, 1997.
18. Borego F, Ulbrecht M, Weiss EH, Coligan JE, Brooks AG: Recognition of human histocompatibility leukocyte antigen (HLA) -E combined with HLA class I signal sequence-derived peptides by CD94/NKG2 confers protection from natural killer cell mediated lysis. J Exp Med 187: 813-818, 1998.
19. Russel TJ, Schultes LM, Kuban DJ: Histocompatibility (HLA) antigens associated with psoriasis. N Eng J Med 287: 738-740, 1972.
20. Asahina A, Akazaki S, Nakagauki H, Kuwata S, Tokunaga K, Ishibashi Y, Juji T: Specific nucleotide sequence of HLA-C is strongly associated with psoriasis vulgaris. J Invest Dermatol 97: 254-258, 1991.
21. Elder JT. The genetics of psoriasis. Arch Dermatol 130: 216-224, 1994.
22. Henseler T. The genetics of psoriasis. J Am Acad Dermatol 37: 1-115, 1994.
23. Trembath RC, Clough RL, Rosbothan JL, Barker JNWB: Identification of a major susceptibility locus on chromosome 6p and evidence for further disease loci revealed by a two stage genome-wide search in psoriasis. Hum Mol Genet 6: 813-818, 1997.
24. Griffiths CEM, Nickoloff BJ,: Induction distribution, and dimunition of leukocyte adhesion molecules (ELAM-1, ICAM-1, VCAM-1), T cell chemotaxin (IL-8) and a modulatory cytokine (TNF-α) during the evolution of allergic contact dermatitis (Rhus dermatitis). Br J Dermatol 124: 519-526, 1991.
25. Griffiths CEM, Voorhees JJ, Nickoloff BJ: Gamma interferon induced different keratinocyte cellular patterns of expression of HLA-DR and DQ and intercellular adhesion molecule-1 (ICAM-1) antigens. Br J Dermatol 120: 1-8, 1989.
26. Boukamp P, Petrusserska RT, Britcreutz D, Hornung J, Marhan A, Fusenig NE: Normal keratinization in a spontaneously immortalized human keratinocyte cell line. J Cell Biol 106: 761-777, 1988.
27. Nickoloff BJ, *et al.*, "Aberrant production of interleukin-8 and thrombospondin-1 by psoriatic keratinocytes mediates angiogenesis," *Am J Pathol*, 144:820-828, 1994.
28. Stem RS: Psoriasis. Lancet 350: 349-353, 1997.
29. Brossay *et al.,* "CD1d mediated recognition of an α-galactosylceramide by natural killer T cells is highly conserved through mammalian evolution," J *Exp Med,* 188(8):1521-1528, 1998.
30. Balk SP, Burke S, Polischuk JE, Frantz ME, Yang L, Porcelli S, Colgan SP, Blumberg RS: β2-microglobulin-independent MHC class Ib molecule expressed by human intestinal epithelium. Science 265: 259-262, 1994.
31. Blumberg RS, Terhorst C, Bleider P, McDermott FV, Allan CH, Landau SB, Torer JS, Balk SP: Expression of a nonpolymorphic MHC class I-like molecule, CD1d, by human intestinal epithelial cells. J Immunol 147: 2518-2524, 1991.
32. Panja A, Blumberg RS, Balk SP, Mayer L: CD1d is involved in T cell-intestinal cell interaction. J Exp Med 178: 1115-1119, 1993.
33. Barker JNW, Mitra RS, Griffith CEM, Dixit VM, Nickoloff BJ: Keratinocytes as initiators of inflammation. Lancet 337:211-214, 1991.
34. Holleran WM, Takagi Y, Menon GK, Legler G, Feingold KR, Elias PM: Processing of epidermal glucosylceramides is required for optimal mammalian cutaneous permeability barrier function. J Clin Invest 91: 1656-1664, 1993.
35. Marsh NL, Elias PM, Holleran WM: Glucosylceramides stimulate murine epidermal hyperproliferation. J Clin Invest 95: 2903-2909, 1995.
36. Wood LC, Jackson SM, Elias PM, Grunfeld C, Feingold KR: Cutaneous barrier perturbation stimulates cytokine production in the epidermis of mice. J Clin Invest 90: 482-487, 1992.
37. Nickoloff BJ, Naidu Y: Perturbation of epidermal barrier function correlates with initiation of cytokine cascade in human skin. J Am Acad Dermatol 30: 535-546, 1994.
38. Haratake A, Uchida Y, Schmuth M, Tanno O, Yasuda R, Epstein JH, Elias PM, Holleran WM: UVB-induced alterations in permeability barrier function: Role for epidermal hyperproliferation and thymocyte-mediated response. J Invest Dermatol 108: 769-775, 1997.
39. Matta S, Monti M, Sesana S, Mellesi L. Ghidani R, Caputo R: Abnormality of water barrier function in psoriasis: role of ceramide fractions. Arch Dermatol 130: 452-456, 1996.
40. Ghadially R, Reed JT, Elias PM: Stratum corneum structure and function correlates with phenotype in psoriasis. J Invest Dermatol 107: 558-564, 1996.
41. Imokawa G, Abe A, Jin K, Higaki Y, Kawashima M, Hidano A: Decreased level of ceramides in stratum corneum of atopic dermatitis: an etiologic factor in atopic dry skin? J Invest Dermatol 96: 523-526, 1991.
42. Friedman SJ: Management of psoriasis vulgaris with a hydrocolloid occlusive dressing. Arch Dermatol 123: 1046-1052, 1987.
43. D'Andrea A, Chang C, Phillips JH, Lanier LL: Regulation of T cell lymphokine modulation by killer cell inhibitory receptor recognition of self - HLA class I alleles. J Exp Med 184: 789-794, 1996.
44. Mallick-Wood CA, Lewis JM, Richie LI, Owen MJ, Tigelaar RE, Hayday AC: Conservation of T Cell receptor conformation in epidermal γδ cells with disrupted primary Vγ gene usage. Science 279: 1729-1733, 1998.

## Claims

1. Use of a peptide, antibody or antibody fragment that inhibits an interaction between a MHC class I-like molecule and CD161 receptor on a NK-T cell for the manufacture of a medicament for treating a cutaneous or extracutaneous disorder, wherein the cutaneous or extracutaneous disorder is selected from the group consisting of psoriasis, atopic dermatitis, allergic/irritant contact dermatitis, lupus erythematosus, lichen planus, drug reactions, alopecia areata and androgenetic alopecia.

## Patentansprüche

1. Verwendung eines Peptids, Antikörpers oder Antikörperfragments, welches/welcher eine Wechselwirkung zwischen einem MHC Klasse I-artigen Molekül und dem CD161-Rezeptor auf einer NK-T-Zelle hemmt, zur Herstellung eines Medikaments zur Behandlung einer Störung der Haut oder der äußeren Haut, wobei die Störung der Haut oder äußeren Haut aus der Gruppe, bestehend aus Psoriasis, Atopischer Dermatits, Allergischer/Gereizter Kontaktdermatitis, *Lupus erythematosus, Lichen planus,* Arzneimittelreaktionen, *Alopecia areata* und androgenetischer Alopezie ausgewählt ist.

## Revendications

1. Utilisation d'un peptide, d'un anticorps ou d'un fragment d'anticorps qui inhibe une interaction entre une molécule CMH de classe I et un récepteur CD 161 sur une cellule NK-T, pour préparer un médicament destiné au traitement d'un trouble cutané ou extracutané, le trouble cutané ou extracutané étant choisi dans l'ensemble consistant en le psoriasis, la dermatite atopique, la dermatite de contact allergique/irritante, le lupus érythémateux, le lichen plan, les réactions médicamenteuses, la pelade et l'alopécie androgénogénétique.
